(19) Europäisches Patentamt
European Patent Office
Office européen des brevets

(11) Veröffentlichungsnummer: **0 354 179 B1**

(12) # EUROPÄISCHE PATENTSCHRIFT

(45) Veröffentlichungstag der Patentschrift: **17.08.94**

(51) Int. Cl.$^5$: **C07D 239/56**, C08K 5/39, C08L 57/08

(21) Anmeldenummer: **89810554.9**

(22) Anmeldetag: **20.07.89**

Die Akte enthält technische Angaben, die nach dem Eingang der Anmeldung eingereicht wurden und die nicht in dieser Patentschrift enthalten sind.

(54) **Thiouracile als Stabilisatoren für chlorhaltige Polymerisate.**

(30) Priorität: **29.07.88 CH 2890/88**

(43) Veröffentlichungstag der Anmeldung:
**07.02.90 Patentblatt 90/06**

(45) Bekanntmachung des Hinweises auf die Patenterteilung:
**17.08.94 Patentblatt 94/33**

(84) Benannte Vertragsstaaten:
**BE DE FR GB IT NL**

(56) Entgegenhaltungen:
**BE-A- 497 785**
**CH-A- 482 402**
**JP-A-76 088 542**
**JP-A-77 049 260**
**US-A- 4 105 627**

**CHEMICAL ABSTRACTS, Band 95, Nr. 6, 10. August 1981, Seite 35, Zusammenfassung Nr. 44163w, Columbus, Ohio, US; & JP-A-56 11 936 (KYODO CHEMICAL CO. LTD) 05-02-1981**

**CHEMICAL ABSTRACTS, Band 70, Nr. 19, 12. Mai 1969, Seite 335, Zusammenfassung Nr. 87730e, Columbus, Ohio, US; K.A: NURIDZHANYAN et al.:"Synthesis of some biologically active derivatives of thiouracil" & KHIM. GETEROTSIKL. SOEDIN., SB. 1: AZOTSODERZHASHCHIE GETEROTSIKLY 1967, 400-3**

(73) Patentinhaber: **CIBA-GEIGY AG**
**Klybeckstrasse 141**
**CH-4002 Basel (CH)**

(72) Erfinder: **Wehner, Wolfgang, Dr.**
**Wetzbach 34**
**D-6144 Zwingenberg (DE)**
Erfinder: **Hartmann, Olaf-René, Dr.**
**Hofgartenstrasse 21**
**D-6140 Bensheim 2 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft mit 2-Thiouracilen gegen thermischen Abbau stabilisierte chlorhaltige Polymerisate und neue 2-Thiouracile.

Es ist bekannt, dass chlorhaltige Polymerisate gegen den schädigenden Einfluss von Licht und Wärme, insbesondere bei der Verarbeitung zu Formteilen, geschützt werden müssen. 2-Thiouracile und deren Verwendung als Stabilisatoren werden beispielsweise in US-A-4 105 627, JP-A-81/11936, JP-A-77/049260 und JP-A-76/088542 offenbart.

Die Verwendung einer Reihe von Uracilen und Thiouracilen als Herbizide ist insbesondere aus FR-A-1 270 771 und CH-A-482 402 bekannt.

Ein mögliches Herstellungsverfahren für Thiouracile wird in Chemical Abstracts 70:87730e (1969) beschrieben.

Ein Gegenstand der vorliegenden Erfindung sind Zusammensetzungen enthaltend a) ein chlorhaltiges Polymerisat und b) mindestens eine Verbindung der Formel I,

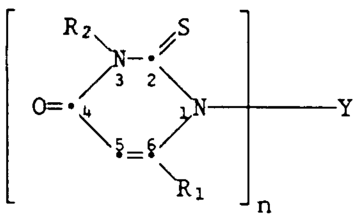

(I)

worin n 1 oder 2 bedeutet, $R_1$ $C_1$-$C_{18}$-Alkyl, $C_3$-$C_{18}$-Alkenyl, Phenyl, $C_7$-$C_{12}$-Phenylalkyl oder am Phenylring durch $C_1$-$C_4$-Alkyl und/oder Chlor substituiertes $C_7$-$C_{12}$-Phenylalkyl ist, $R_2$ $C_1$-$C_{18}$-Alkyl, eine Gruppe

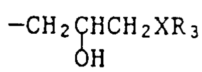

oder durch Hydroxy oder eine Gruppe -$XR_3$ substituiertes $C_2$-$C_{22}$-Alkyl ist, wobei X Sauerstoff oder Schwefel ist und $R_3$ $C_1$-$C_{18}$-Alkyl, Phenyl oder $C_7$-$C_{12}$-Phenylalkyl darstellt, oder $R_2$ ferner $C_3$-$C_{18}$-Alkenyl,$C_7$-$C_{12}$-Phenylalkyl, am Phenylring durch $C_1$-$C_4$-Alkyl und/oder Chlor substituiertes $C_7$-$C_{12}$-Phenylalkyl, Di($C_1$-$C_4$-alkyl)thiocarbamoyl oder eine Gruppe der Formel IIa oder IIb ist,

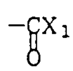 , 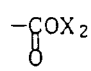

(IIa) (IIb)

wobei $X_1$ $C_1$-$C_{17}$-Alkyl, $C_3$-$C_{17}$-Alkenyl, $C_5$-$C_7$-Cycloalkyl, durch $C_1$-$C_4$-Alkyl substituiertes $C_5$-$C_7$-Cycloalkyl, Phenyl, durch $C_1$-$C_4$-Alkyl und/oder Chlor substituiertes Phenyl, $C_7$-$C_{12}$-Phenylalkyl, am Phenylring durch $C_1$-$C_4$-Alkyl und/oder Chlor substituiertes $C_7$-$C_{12}$-Phenylalkyl, $C_1$-$C_{10}$-Alkylthio oder Di($C_1$-$C_4$-alkyl)-amino bedeutet und $X_2$ $C_1$-$C_{18}$-Alkyl, $C_3$-$C_{18}$-Alkenyl, $C_5$-$C_7$-Cycloalkyl, durch $C_1$-$C_4$-Alkyl substituiertes $C_5$-$C_7$-Cycloalkyl, Phenyl, durch $C_1$-$C_4$-Alkyl und/oder Chlor substituiertes Phenyl, $C_7$-$C_{12}$-Phenylalkyl oder am Phenylring durch $C_1$-$C_4$-Alkyl und/oder Chlor substituiertes $C_7$-$C_{12}$-Phenylalkyl ist, wenn n 1 bedeutet, Y Di($C_1$-$C_4$-alkyl)thiocarbamoyl oder eine Gruppe der Formel IIa oder IIb ist, wenn n 2 bedeutet, Y eine Gruppe IIc oder IId ist,

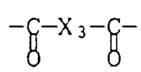 , 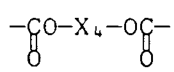

(IIc) (IId)

wobei $X_3$ $C_1$-$C_{12}$-Alkylen oder Phenylen bedeutet und $X_4$ $C_2$-$C_{12}$-Alkylen oder durch 1 oder 2 Sauerstoffatome unterbrochenes $C_4$-$C_8$-Alkylen ist, mit der Massgabe, dass die Reste Y und $R_2$ gleich sind, wenn $R_2$ Di($C_1$-$C_4$-alkyl)thiocarbamoyl oder eine Gruppe der Formel IIa oder IIb bedeutet.

Alkyl mit bis zu 18 Kohlenstoffatomen bedeutet beispielsweise Methyl, Ethyl, Propyl, n-Butyl, i-Butyl, t-Butyl, Pentyl, Hexyl, Heptyl, n-Octyl, i-Octyl, i-Nonyl, n-Decyl, n-Dodecyl oder n-Octadecyl. $R_1$ ist bevorzugt $C_1$-$C_4$-Alkyl, insbesondere Methyl. Eine der bevorzugten Bedeutungen von $R_2$ ist $C_1$-$C_{12}$-Alkyl, zum Beispiel $C_1$-$C_4$-Alkyl oder $C_4$-$C_{12}$-Alkyl.

Alkenyl mit bis zu 18 Kohlenstoffatomen bedeuten zum Beispiel Allyl, 2-Methallyl, Hexenyl, Decenyl, Undecenyl, Heptadecenyl oder Oleyl. Bevorzugte Bedeutungen für $R_2$ als Alkenyl sind z.B. Allyl und Oleyl. In den Alkenylresten ist das Kohlenstoffatom in der Stellung 1 bevorzugt ein gesättigtes C-Atom.

Beispiele für $C_7$-$C_{12}$-Phenylalkyl, welches gegebenenfalls am Phenylring durch $C_1$-$C_4$-Alkyl und/oder Chlor substituiert sein kann, sind Benzyl, 2-Phenylethyl, Methylbenzyl, Dimethylbenzyl, t-Butylbenzyl und Chlorbenzyl. Benzyl ist bevorzugt.

Die Gruppe

$$-CH_2\underset{OH}{CH}CH_2XR_3$$

bedeutet bevorzugt 3-(2-Ethylhexyl)oxy-2-hydroxypropyl oder 3-(2-Ethylhexyl)thio-2-hydroxypropyl.

Durch Hydroxy oder eine Gruppe $-XR_3$ substituiertes $C_2$-$C_{22}$-Alkyl, insbesondere $C_2$-$C_{12}$-Alkyl, bedeutet beispielsweise 2-Hydroxyethyl, 2-Methoxyethyl, 2-Hydroxypropyl, 2,3-Dihydroxypropyl, 9,10-Dihydroxystearyl oder 2,3,4,5,6-Pentahydroxyhexyl. Durch Hydroxy oder $C_1$-$C_6$-Alkyloxy substituiertes $C_2$-$C_6$-Alkyl ist bevorzugt. 2-Hydroxyethyl und 2-Methoxyethyl sind besonderes bevorzugt. Die OH-Gruppe bzw. die Gruppe $-XR_3$ befinden sich bevorzugt nicht in 1-Stellung.

Di($C_1$-$C_4$-alkyl)thiocarbamoyl bedeutet zum Beispiel folgende Gruppen:

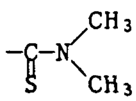 , 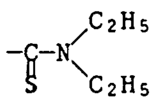 , 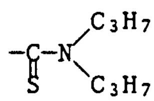 , 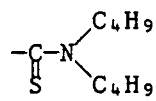 .

$C_5$-$C_7$-Cycloalkyl, welches gegebenenfalls durch $C_1$-$C_4$-Alkyl substituiert ist, bedeutet zum Beispiel Cyclopentyl, Cyclohexyl, Cycloheptyl, Methylcyclohexyl oder t-Butylcyclohexyl. Cyclohexyl ist bevorzugt.

Phenyl, welches durch $C_1$-$C_4$-Alkyl und/oder Chlor substituiert ist, bedeutet zum Beispiel Chlorphenyl, 2,4-Dichlorphenyl, Trichlorphenyl, Methylphenyl, Dimethylphenyl, 2-Methyl-6-ethylphenyl, 2-Methyl-6-tert-butylphenyl, 4-tert-Butylphenyl oder 3-Chlor-2-methylphenyl.

Beispiele für $C_1$-$C_{10}$-Alkylthio sind Methylthio, Ethylthio, Propylthio, Butylthio, Pentylthio, Hexylthio, Heptylthio, Octylthio, Nonylthio und Decylthio. Octylthio ist bevorzugt.

Di($C_1$-$C_4$-alkyl)amino bedeutet zum Beispiel Dimethylamino, Diethylamino, Dipropylamino und Dibutylamino. Dimethylamino und Diethylamino sind bevorzugt.

Beispiele für Alkylen mit bis zu 12 Kohlenstoffatomen sind Methylen, Dimethylen, Trimethylen, Tetramethylen, Pentamethylen, Hexamethylen, Heptamethylen, Octamethylen, Nonamethylen, Decamethylen und Dodecamethylen.

$C_4$-$C_8$-Alkylen, welches durch 1 oder 2 Sauerstoffatome unterbrochen ist, bedeutet zum Beispiel 3-Oxapentamethylen oder 3,6-Dioxaoctamethylen.

Bevorzugt sind Zusammensetzungen, worin n 1 bedeutet, $R_2$ $C_1$-$C_{18}$-Alkyl, eine Gruppe

$$-CH_2\underset{OH}{CH}CH_2XR_3,$$

durch Hydroxy oder eine Gruppe $-XR_3$ substituiertes $C_2$-$C_{22}$-Alkyl, $C_3$-$C_{18}$-Alkenyl, $C_7$-$C_{12}$-Phenylalkyl oder am Phenylring durch $C_1$-$C_4$-Alkyl und/oder Chlor substituiertes $C_7$-$C_{12}$-Phenylalkyl ist und Y Wasserstoff bedeutet.

Von Interesse sind auch Zusammensetzungen, worin n 1 ist und Y Di($C_1$-$C_4$-alkyl)thiocarbamoyl oder eine Gruppe IIa oder IIb bedeutet.

Besonders bevorzugt sind Zusammensetzungen, worin n 1 ist, $R_1$ $C_1$-$C_4$-Alkyl oder Phenyl bedeutet, $R_2$ $C_1$-$C_{18}$-Alkyl, durch Hydroxy oder $C_1$-$C_6$-Alkyloxy substituiertes $C_2$-$C_6$-Alkyl, $C_3$-$C_{18}$-Alkenyl, Benzyl oder Benzoyl ist, Y Di($C_1$-$C_4$-alkyl)thiocarbamoyl oder eine Gruppe IIa oder IIb ist, $X_1$ $C_1$-$C_{12}$-Alkyl, Phenyl,

Dichlorphenyl, $C_1$-$C_{10}$-Alkylthio oder Di($C_1$-$C_4$-alkyl)amino bedeutet und $X_2$ $C_1$-$C_{12}$-Alkyl darstellt.

Von besonderem Interesse sind auch Zusammensetzungen, worin n 1 ist, $R_1$ Methyl bedeutet, $R_2$ $C_1$-$C_4$-Alkyl, 2-Hydroxyethyl, 2-Methoxyethyl oder Allyl ist und Y Benzoyl oder ($C_1$-$C_{12}$-Alkyl)oxycarbonyl bedeutet.

$R_1$ bedeutet bevorzugt $C_1$-$C_4$-Alkyl oder Phenyl.

$R_2$ bedeutet bevorzugt $C_1$-$C_{18}$-Alkyl, durch Hydroxy oder $C_1$-$C_6$-Alkyloxy substituiertes $C_2$-$C_6$-Alkyl, $C_3$-$C_{18}$-Alkenyl, Benzyl oder Benzoyl.

Ein weiterer Gegenstand der Erfindung sind Zusammensetzungen enthaltend a) ein chlorhaltiges Polymerisat, b) mindestens eine Verbindung der Formel I oder IA

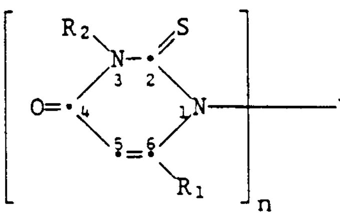

(IA)

worin $R_1$ $C_1$-$C_4$-Alkyl oder Phenyl bedeutet und $R_2$ $C_1$-$C_{18}$-Alkyl, durch Hydroxy oder $C_1$-$C_6$-Alkyloxy substituiertes $C_2$-$C_6$-Alkyl, $C_3$-$C_{18}$-Alkenyl, Benzyl oder Benzoyl ist, und als zusätzliche Komponente c) eine Epoxyverbindung.

Die Verbindung der Formel IA ist bevorzugt 3,6-Dimethyl-2-thiouracil.

Bei der Epoxyverbindung handelt es sich bevorzugt um epoxidierte Oele und epoxidierte Fettsäureester, z.B. epoxidiertes Sojabohnenöl und epoxidiertes Butyloleat.

Bei den chlorhaltigen Polymerisaten handelt es sich bevorzugt um Vinylchloridhomopolymere oder -copolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS. Bevorzugte Substrate sind auch Mischungen der vorstehend genannten Homo- und Copolymerisate, insbesondere Vinylchlorid-Homopolymerisate, mit anderen thermoplastischen oder/und elastomeren Polymeren, insbesondere mit ABS, MBS, NBR, SAN, EVA.

Weiterhin bevorzugt sind Suspensions- und Massepolymere sowie Emulsionspolymere.

Als chlorhaltiges Polymerisat ist Polyvinylchlorid besonders bevorzugt.

Es ist vorteilhaft, die Verbindungen der Formel I oder IA zusammen mit bekannten Thermostabilisatoren einzusetzen, wie z.B. Organozinnverbindungen, Bleiverbindungen, organischen Antimonverbindungen, Me(II)-Phenolaten, insbesondere $C_7$-$C_{20}$-Alkylphenolaten, beispielsweise Nonylphenolat, oder Me(II)-Carboxylaten. Me(II) bedeutet z.B. Ba, Ca, Mg, Cd oder Zn. Bei den Carboxylaten handelt es sich bevorzugt um Salze von Carbonsäuren mit 7 bis 20 C-Atomen, beispielsweise Benzoate, Alkenoate oder Alkanoate, bevorzugt Stearate, Oleate, Laurate, Palmitate, Hydroxystearate oder 2-Ethylhexanoate. Besonders bevorzugt sind Stearate, Oleate und p-tert-Butylbenzoate. Beispiele für Organozinnverbindungen, Bleiverbindungen und organische Antimonverbindungen sind die in US-A-4 743 640 Spalte 3, Zeile 48 bis Spalte 5, Zeile 38 genannten Verbindungen.

Zusätzlich können die mit den Verbindungen der Formel I oder IA stabilisierten chlorhaltigen Polymerisate in üblichen Mengen herkömmliche PVC-Stabilisatoren enthalten, wie beispielsweise Phosphite, bevorzugt solche der Formeln

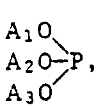 , 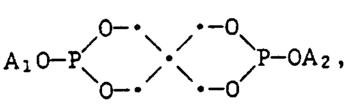 , 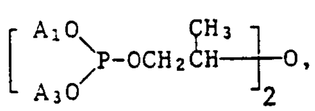 , 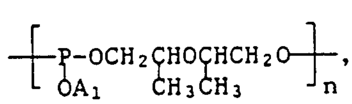 ,

worin $A_1$, $A_2$ und $A_3$ unabhängig voneinander $C_4$-$C_{18}$-Alkyl, $C_6$-$C_{18}$-Alkenyl, $C_5$-$C_7$-Cycloalkyl, Phenyl oder durch ein bis drei $C_1$-$C_{12}$-Alkylgruppen substituiertes Phenyl bedeuten.

Beispiele sind Trioctyl-, Tridecyl-, Tridodecyl-, Tritetradecyl-, Tristearyl-, Trioleyl-, Triphenyl-, Trikresyl-, Tris-p-nonylphenyl- und Tricyclohexylphosphit. Bevorzugt sind die Aryldialkyl- sowie die Alkyldiarylphosphite, wie z.B. Phenyldidecyl-, (2,4-Di-tert-butylphenyl)didodecyl-, (2,6-Di-tert-butylphenyl)didodecylphosphit und die Dialkyl-und Diaryl-pentaerythrit-diphosphite, wie z.B. Distearylpentaerythrit-diphosphit.

Ebenfalls bevorzugt sind die Tetraphenyl- und Tetraalkyl-[dipropylenglykol-1,2]-diphosphite und die Poly-[dipropylenglykol-1,2-phenylphosphite] sowie die Poly-[dipropylenglykol-1,2-alkylphosphite]. Besonders bevorzugte organische Phosphite sind Distearyl-pentaerythritdiphosphit, Tris(nonylphenyl)phosphit, Phenyldidecylphosphit, Tetraphenyl-[dipropylenglykol-1,2]-diphosphit und Poly-[dipropylenglykol-1,2-phenylphosphit].

Ein bevorzugter Gegenstand der Erfindung sind daher Zusammensetzungen enthaltend ausser der Komponente a) und einer Verbindung der Formel I mindestens ein Me(II)-Carboxylat und/oder Me(II)-Phenolat, wobei Me(II) Ba, Ca, Mg, Cd oder Zn bedeutet, und gegebenenfalls ein Phosphit.

Gemäss einer weiteren Bevorzugung enthalten die erfindungsgemässen Zusammensetzungen ausser der Komponente a) und einer Verbindung der Formel I mindestens ein Me(II)-Carboxylat, wobei Me(II) Ba, Ca, Mg oder Zn bedeutet. Gemische aus Ba/Zn- oder Ca/Zn-Carboxylaten werden dabei als Costabilisatoren besonders bevorzugt.

Bevorzugt sind auch Zusammensetzungen enthaltend ausser der Komponente a), einer Verbindung der Formel IA und der Komponente c) mindestens ein Me(II)-Carboxylat und/oder Me(II)-Phenolat, wobei Me(II), Ba, Ca, Mg, Cd oder Zn bedeutet, und gegebenenfalls ein Phosphit, insbesondere ein solches der oben angegebenen Formeln.

Die bekannten Thermostabilisatoren (z.B. Carboxylate) können in dem zu stabilisierenden Material in einer dem Fachmann bekannten Konzentration vorliegen, wie zum Beispiel in Mengen von 0,05 bis 5 Gew.%.

Die Phosphite werden z.B. in Konzentrationen von 0,3 bis 5, vorzugsweise 0,5 bis 1 Gew.% und die Epoxyverbindungen, wie z.B. das epoxidierte Sojabohnenöl, in Konzentrationen von 1 bis 8, vorzugsweise 1 bis 3 Gew.% eingesetzt.

Die Verbindungen der Formel I bzw. IA werden beispielsweise in Mengen von 0,05 bis 5, bevorzugt 0,05 bis 1, insbesondere 0,1 bis 0,5 Gew.% in das chlorhaltige Polymerisat eingearbeitet.

Die Angabe Gew.% bezieht sich jeweils auf das zu stabilisierende Material.

Je nach dem Verwendungszweck der Polymerisate können vor oder bei der Einarbeitung der Stabilisatoren auch weitere Zusätze eingearbeitet werden, wie zum Beispiel phenolische Antioxidantien, Gleitmittel (bevorzugt Montanwachse oder Glycerinester), Fettsäureester, Paraffine, Weichmacher, Füllstoffe, Russ, Asbest, Kaolin, Talk, Glasfasern, Modifikatoren (wie etwa Schlagzäh-Zusätze), optische Aufheller, Pigmente, Lichtschutzmittel, UV-Absorber, Flammschutzmittel oder Antistatika.

Weitere mögliche Zusätze sind ferner β-Aminocrotonate, z.B. die in DE-A-804 442, DE-A-807 207 und JP-A-75/17454 beschriebenen Verbindungen, Pyrrole, z.B. die in EP-A-22 087 angegebenen Verbindungen, Aminouracile, z.B. die in EP-A-65 934 offenbarten Verbindungen, Aminothiouracile, z.B. die aus EP-A-41 479 bekannten Verbindungen, Polyole, z.B. die in DE-A-3 019 910 beschriebenen Verbindungen, β-Diketone, z.B die in DE-A-2 600 516 angegebenen Verbindungen, oder auch Gemische aus β-Diketonen und Hydrotalciten, wie z.B. in EP-A-63 180 beschrieben.

Die Einarbeitung der Stabilisatorkomponenten in das chlorhaltige Polymerisat erfolgt am günstigsten, wie üblich, auf einem Mischwalzwerk, z.B. einem 2-Walzenstuhl bei Temperaturen zwischen 150° und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Ein weiterer Gegenstand der Erfindung sind die neuen Verbindungen der Formeln IIIA, IIIB, IIIC, IIID und IIIE,

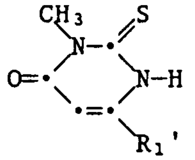

(IIIA)

worin $R_1'$ $C_3$-$C_{18}$-Alkyl, $C_3$-$C_{18}$-Alkenyl, $C_7$-$C_{12}$-Phenylalkyl oder am Phenylring durch $C_1$-$C_4$-Alkyl und/oder Chlor substituiertes $C_7$-$C_{12}$-Phenylalkyl darstellt;

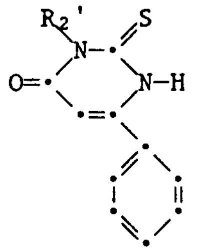

(IIIB),

worin $R_2'$ $C_1$-$C_{18}$-Alkyl, $C_3$-$C_{18}$-Alkenyl, $C_7$-$C_{12}$-Phenylalkyl oder am Phenylring durch $C_1$-$C_4$-Alkyl und/oder Chlor substituiertes $C_7$-$C_{12}$-Phenylalkyl darstellt;

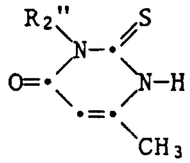

(IIIC),

worin $R_2''$ $C_{10}$-$C_{18}$-Alkyl oder $C_{10}$-$C_{18}$-Alkenyl ist;

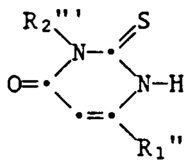

(IIID),

worin $R_1''$ $C_1$-$C_{18}$-Alkyl, $C_3$-$C_{18}$-Alkenyl, Phenyl, $C_7$-$C_{12}$-Phenylalkyl oder am Phenylring durch $C_1$-$C_4$-Alkyl und/oder Chlor substituiertes $C_7$-$C_{12}$-Phenylalkyl bedeutet und $R_2'''$ eine Gruppe

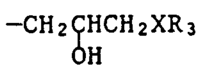

oder durch Hydroxy oder eine Gruppe $-XR_3$ substituiertes $C_2$-$C_{22}$-Alkyl darstellt, wobei X Sauerstoff oder Schwefel ist und $R_3$ $C_1$-$C_{18}$-Alkyl, Phenyl oder $C_7$-$C_{12}$-Phenylalkyl ist;

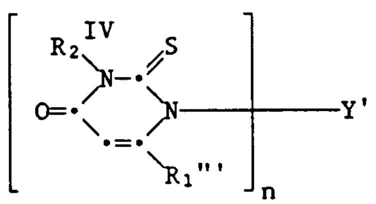

(IIIE),

worin n 1 oder 2 bedeutet, $R_1'''$ $C_1$-$C_{18}$-Alkyl, $C_3$-$C_{18}$-Alkenyl, Phenyl, $C_7$-$C_{12}$-Phenylalkyl oder am Phenylring durch $C_1$-$C_4$-Alkyl und/oder Chlor substituiertes $C_7$-$C_{12}$-Phenylalkyl ist, $R_2^{IV}$ $C_1$-$C_{18}$-Alkyl, eine Gruppe

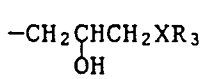

oder durch Hydroxy oder eine Gruppe $-XR_3$ substituiertes $C_2$-$C_{22}$-Alkyl ist, wobei X Sauerstoff oder Schwefel bedeutet und $R_3$ $C_1$-$C_{18}$-Alkyl, Phenyl oder $C_7$-$C_{12}$-Phenylalkyl ist, oder $R_2$ ferner $C_3$-$C_{18}$-Alkenyl, $C_7$-$C_{12}$-Phenylalkyl, am Phenylring durch $C_1$-$C_4$-Alkyl und/oder Chlor substituiertes $C_7$-$C_{12}$-Phenylalkyl, Di-

($C_1$-$C_4$-alkyl)thiocarbamoyl oder eine Gruppe der Formel IIa oder IIb ist,

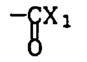 , 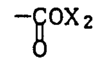

(IIa) (IIb)

wobei $X_1$ $C_1$-$C_{17}$-Alkyl, $C_3$-$C_{17}$-Alkenyl, $C_5$-$C_7$-Cycloalkyl, durch $C_1$-$C_4$-Alkyl substituiertes $C_5$-$C_7$-Cycloalkyl, Phenyl, durch $C_1$-$C_4$-Alkyl und/oder Chlor substituiertes Phenyl, $C_7$-$C_{12}$-Phenylalkyl, am Phenylring durch $C_1$-$C_4$-Alkyl und/oder Chlor substituiertes $C_7$-$C_{12}$-Phenylalkyl, $C_1$-$C_{10}$-Alkylthio oder Di($C_1$-$C_4$-alkyl)-amino bedeutet und $X_2$ $C_1$-$C_{18}$-Alkyl, $C_3$-$C_{18}$-Alkenyl, $C_5$-$C_7$-Cycloalkyl, durch $C_1$-$C_4$-Alkyl substituiertes $C_5$-$C_7$-Cycloalkyl, Phenyl, durch $C_1$-$C_4$-Alkyl und/oder Chlor substituiertes Phenyl, $C_7$-$C_{12}$-Phenylalkyl oder am Phenylring durch $C_1$-$C_4$-Alkyl und/oder Chlor substituiertes $C_7$-$C_{12}$-Phenylalkyl ist, wenn n 1 bedeutet, Y′ Di($C_1$-$C_4$-alkyl)thiocarbamoyl oder eine Gruppe der Formel IIa oder IIb ist, wenn n 2 bedeutet, Y′ eine Gruppe IIc oder IId ist,

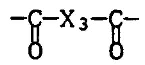 , 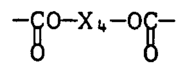

(IIc) (IId)

wobei $X_3$ $C_1$-$C_{12}$-Alkenyl oder Phenylen bedeutet und $X_4$ $C_2$-$C_{12}$-Alkylen oder durch 1 oder 2 Sauerstoffatome unterbrochenes $C_4$-$C_8$-Alkylen ist, mit der Massgabe, dass $R_2^{IV}$ und Y′ gleich sind, wenn $R_2^{IV}$ Di($C_1$-$C_4$-alkyl)thiocarbamoyl oder eine Gruppe der Formel IIa oder IIb ist.

Beispiele für $R_1'$, $R_1''$, $R_1'''$, $R_2'$, $R_2''$, $R_2'''$, $R_2^{IV}$ und Y′ sind die unter der Formel I angegebenen Bedeutungen.

Bevorzugt sind Verbindungen der Formeln IIIA, IIIB und IIIC, worin $R_1'$ $C_3$-$C_4$-Alkyl bedeutet, $R_2'$ $C_1$-$C_{18}$-Alkyl, Allyl oder Benzyl darstellt und $R_2''$ $C_{10}$-$C_{18}$-Alkyl oder Allyl ist.

Ebenfalls bevorzugt sind Verbindungen der Formel IIID, worin $R_1''$ $C_1$-$C_4$-Alkyl oder Phenyl bedeutet und $R_2'''$ durch Hydroxy oder $C_1$-$C_6$-Alkyloxy substituiertes $C_2$-$C_6$-Alkyl ist.

Von Interesse sind auch Verbindungen der Formel IIIE, worin n 1 bedeutet, $R_1'''$ $C_1$-$C_4$-Alkyl ist, $R_2^{IV}$ $C_1$-$C_4$-Alkyl, Allyl, Benzyl oder Benzoyl bedeutet und Y' Di($C_1$-$C_4$-alkyl)thiocarbamoyl oder eine Gruppe IIa oder IIb ist, $X_1$ $C_1$-$C_{12}$-Alkyl, Phenyl, Dichlorphenyl, $C_1$-$C_{10}$-Alkylthio oder Di($C_1$-$C_4$-alkyl)amino bedeutet und $X_2$ $C_1$-$C_{12}$-Alkyl darstellt.

Die Verbindungen der Formel I können in Analogie zu bekannten Verfahren hergestellt werden.

Ein mögliches Herstellungsverfahren für Verbindungen der Formel I, worin Y Wasserstoff bedeutet, wird durch folgende Reaktionsgleichung beschrieben:

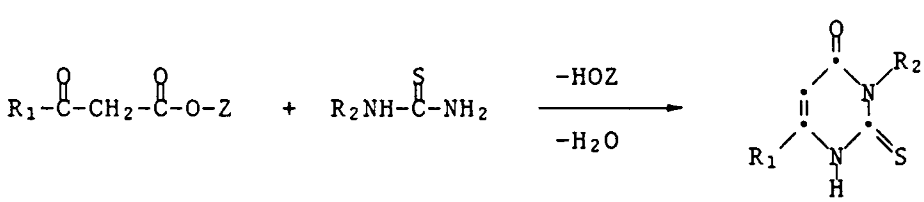


(IV) (V) (I)

Z ist z.B. $C_1$-$C_4$-Alkyl, insbesondere Methyl oder Ethyl. Die Umsetzung findet zweckmässigerweise unter Rückflussbedingungen in Gegenwart einer starken Base in einem organischen Lösungsmittel, bevorzugt einem kurzkettigen Alkohol, insbesondere Methanol oder Ethanol, statt. Die Umsetzung kann z.B. auch in IV als Reaktionsmedium bei 60-120 °C durchgeführt werden. Bei den Basen handelt es sich beispielsweise um Alkalialkoholate oder Alkalihydroxide, bevorzugt Natriummethylat, Natriumethylat, Natriumhydroxid oder Kaliumhydroxid. Die Reaktanden können z.B. in stöchiometrischem Verhältnis eingesetzt werden; bevorzugt ist ein Molverhältnis von IV/V/Base = (1,0-2,5)/1/(1,0-3,0). Nach Beendigung der Umsetzung erfolgt die Isolierung des Produktes sowie seine Reinigung nach üblichen Methoden.

Die Ausgangsprodukte der Formeln IV und V sind im Handel erhältlich oder können in Analogie zu bekannten Verfahren hergestellt werden. Die Verbindung der Formel V kann z.B. gemäss folgender Reaktion erhalten werden:

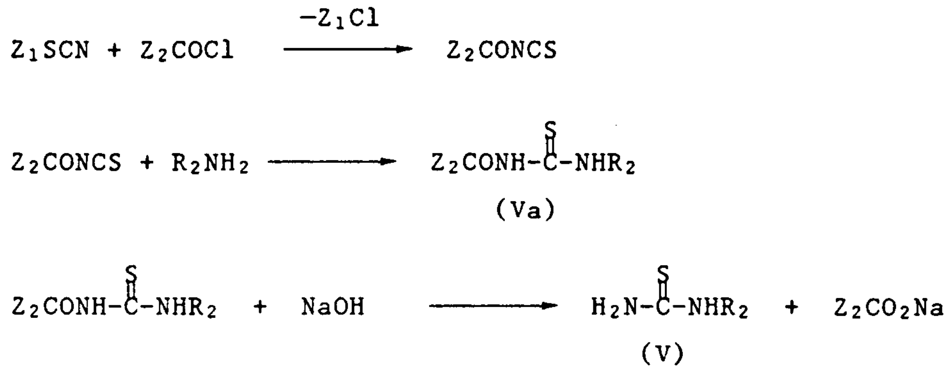

(Va)

(V)

$Z_1$ bedeutet z.B. Kalium, Natrium oder Ammonium und $Z_2$ ist z.B. Methoxy, Ethoxy oder Phenyl. Die Umsetzung findet zweckmässigerweise in einem organischen Lösungsmittel, bevorzugt Aceton oder Essigester, statt. Es ist auch möglich, die Verbindung der Formel Va ohne Abspaltung der Schutzgruppe $Z_2$CO- direkt für die Herstellung der Verbindungen der Formel I zu verwenden.

Die Verbindungen der Formel I, die in 1 oder 1 und 3 Stellung acyliert sind, lassen sich ebenfalls in Analogie zu bekannten Verfahren herstellen; beispielsweise durch Umsetzung des Natriumsalzes des entsprechenden Thiouracils mit einer Acylchlorverbindung.

Einige Verbindungen der Formel I können bei der Herstellung in einem Isomerengemisch anfallen, welches nach üblichen, dem Fachmann bekannten Verfahren aufgetrennt werden kann.

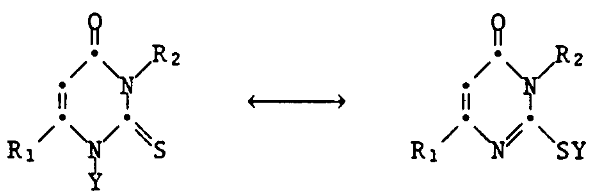

(I) (I*)

Die Formel I ist daher so zu verstehen, dass sie auch die Verbindungen der Formel I* umfasst.

Die folgenden Beispiele erläutern die Erfindung weiter. Teile- und Prozentangaben beziehen sich darin, soweit nichts anderes angegeben ist, auf das Gewicht.

Beispiel 1: Herstellung von 3,6-Dimethyl-2-thiouracil.

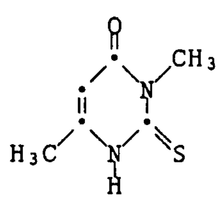

Zu einer Lösung von 216 g (1,2 Mol) 30%igem Natriummethylat in 200 ml abs. Methanol werden 116,1 g (1 Mol) Methylacetoacetat getropft.

Anschliessend werden portionsweise 90,2 g (1 Mol) N-Methylthioharnstoff unter Rühren hinzugegeben. Das Reaktionsgemisch wird 3 Stunden am Rückfluss erhitzt, wobei die anfangs klare Lösung allmählich dickflüssiger wird. Nach Beendigung der Reaktion wird das Methanol im Vakuum abgedampft und der Rückstand in 500 ml Wasser aufgenommen. Das erhaltene Gemisch wird mit konzentrierter Salzsäure neutralisiert, wobei $CO_2$ frei wird. Anschliessend wird der pH-Wert auf 4-5 mit 40 ml Eisessig eingestellt.

Der entstandene Niederschlag wird abgesaugt, mit einem Wasser/Methanol Gemisch (2:1) gewaschen und getrocknet. Das Produkt besitzt einen Schmelzpunkt von 276 °C. Die Ausbeute beträgt 122,5 g (≙78,4 % der Theorie).

Beispiele 2-6: Die in Tabelle 1 angegebenen Verbindungen werden in Analogie zu dem in Beispiel 1 beschriebenen Verfahren hergestellt.

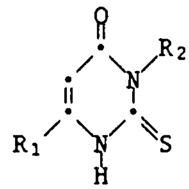

Tabelle 1:

| Bsp. | $R_1$ | $R_2$ | Schmelzpunkt |
|---|---|---|---|
| 2 | $-C_3H_7$-n | $-CH_3$ | 162°C (nach Umfällen aus Methylenchlorid/Isopropylether) |
| 3 | $-C_4H_9$-t | $-CH_3$ | 156°C (nach Umfällen aus Methylenchlorid/Isopropylether) |
| 4 | $-CH_3$ | $-C_2H_5$ | 206°C (nach Digerieren mit Methanol) |
| 5 | $-CH_3$ | $-CH_2-CH=CH_2$ | 185°C (nach Waschen mit $H_2O$) |
| 6 | $-CH_3$ | $-CH_2-$ 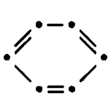 | 215°C (nach Waschen mit $H_2O$ und anschliessender Umkristallisation aus DMF/Ethanol) |

Beispiel 7: Herstellung von 3-n-Octyl-6-methyl-2-thiouracil.

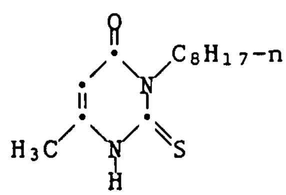

A) Herstellung von N-Benzoyl-N'-n-octylthioharnstoff.

150,7 g (1,55 Mol) Kaliumthiocyanat werden unter Rühren zu 0,8 l Aceton gegeben. Anschliessend werden bei Raumtemperatur 203,9 g (1,45 Mol) Benzoylchlorid zugetropft. Das erhaltene Gemisch wird 10 Minuten am Rückfluss erhitzt. Dann werden 187,5 g (1,45 Mol) n-Octylamin in 0,2 l Aceton so zugetropft, dass der Rückfluss erhalten bleibt (exotherme Reaktion). Nach Beendigung der Reaktion wird das Aceton bei Normaldruck abdestilliert. Der hochviskose Rückstand (535,0 g) besteht aus N-Benzoyl-N'-n-octylthioharnstoff (421,1 g), Kaliumchlorid und etwas Kaliumthiocyanat. Der Rückstand kann ohne Aufarbeitung weiterverwendet werden.

B) Herstellung von 3-n-Octyl-6-methyl-2-thiouracil.

535,0 g des gemäss A) erhaltenen Rückstands werden zu 0,5 l absolutem Methanol gegeben und 5 Minuten am Rückfluss erhitzt. Anschliessend werden in der Siedehitze 576,0 g einer 30%igen Natriummethylatlösung und 2,7 g Wasser zugetropft (exotherme Reaktion) und das Gemisch weitere 10 Minuten gekocht. Dann werden 174,2 g (1,5 Mol) Methylacetoacetat in 0,5 l absolutem Methanol zugetropft (leicht exotherme Reaktion) und das Reaktionsgemisch wird 20 Stunden am Rückfluss erhitzt. Nach Beendigung der Reaktion werden das Methanol sowie das Reaktionswasser abdestilliert. Der Rückstand wird mit 0,5 l 5%iger NaOH versetzt und 30 Minuten auf 50 °C unter Rühren erhitzt. Anschliessend wird das Gemisch bei Raumtemperatur mit 3,0 l Wasser verdünnt und der erhaltene Niederschlag (109,0 g n-Octylthioharnstoff) wird abgesaugt. Das stark alkalische Filtrat wird mit konzentrierter Salzsäure angesäuert ($CO_2$-Entwicklung). Der gebildete Niederschlag wird abgesaugt, chloridfrei gewaschen und bis zur Gewichtskonstanz getrocknet. Das erhaltene 3-n-Octyl-6-methyl-2-thiouracil besitzt einen Schmelzpunkt von 142 °C.

Beispiel 8: Herstellung von 3-(2-Hydroxyethyl)-6-methyl-2-thiouracil.

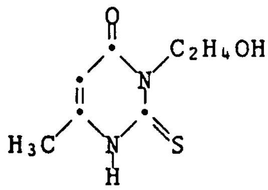

27,9 g (0,232 Mol) N-(2-Hydroxyethyl)thioharnstoff werden in 100 ml absolutem Methanol gelöst. Unter Rühren werden 29,0 g (0,25 Mol) Methylacetoacetat und 50,4 g (0,28 Mol) einer 30%igen methanolischen Natriummethylatlösung zugegeben. Nach 6stündigem Erhitzen am Rückfluss wird das Methanol abdestilliert, der Rückstand in 150 ml Wasser gelöst und mit konzentrierter Salzsäure angesäuert. Der erhaltene Niederschlag wird abgesaugt, neutral gewaschen und bis zur Gewichtskonstanz getrocknet. Das Produkt besitzt einen Schmelzpunkt von 200 °C. Die Ausbeute beträgt 23,0 g (≙53 % der Theorie).

Beispiel 9: Herstellung von 3-(n-Butyl)-6-methyl-2-thiouracil.

Zu einer Lösung von 0,1 Mol N-(n-Butyl)thioharnstoff und 0,11 Mol Methylacetoacetat in 100 ml absolutem Methanol werden unter Rühren 0,125 Mol einer 30%igen Natriummethylatlösung getropft. Nach 8stündigem Kochen am Rückfluss werden weitere 0,11 Mol Methylacetoacetat und 0,125 Mol Natriummethylatlösung hinzugegeben. Dann wird das Reaktionsgemisch weitere 16 Stunden am Rückfluss erhitzt. Anschliessend wird das Methanol abdestilliert und der Rückstand in 200 ml Wasser aufgenommen. Die Lösung wird mit konzentrierter Salzsäure angesäuert, der erhaltene Niederschlag abgesaugt, neutral gewaschen und bis zur Gewichtskonstanz getrocknet. Das Produkt besitzt einen Schmelzpunkt von 164 °C. Die Ausbeute beträgt 80,8 % der Theorie.

Beispiel 10-16: Die in Tabelle 2 angegebenen Verbindungen werden in Analogie zu dem in Beispiel 9 beschriebenen Verfahren hergestellt und aufgearbeitet, soweit nichts anderes vermerkt ist.

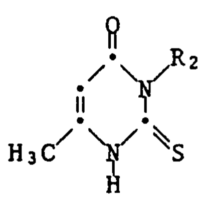

Tabelle 2:

| Bsp. | $R_2$ | Reaktanden | | | Reaktionszeit in Stunden | Ausbeute / Schmelzpunkt |
|---|---|---|---|---|---|---|
| | | Thioharnstoff (Mol) | Methylacetoacetat (Mol) | NaOMe (Mol) | | |
| 10 | $-C_8H_{17}$-i [1] | i-$H_{17}C_8NHCSNH_2$ 0,52 | 1,2 [2] | 1,2 [2] | 10 | <br>154°C (nach Umkristallisation aus einem Petrolether/-i-Propylether Gemisch) |
| 11 | $-C_{10}H_{21}$-n | n-$H_{21}C_{10}NHCSNH_2$ 0,1 | 0,22 [3] | 0,25 [3] | 24 | 93 %<br>124°C |
| 12 [4] | $-C_{12}H_{25}$-n | n-$H_{25}C_{12}NHCSNH_2$ 0,1 | 0,22 [3] | 0,25 [3] | 24 | 83,5 %<br>126°C |
| 13 [7] | $-C_9H_{19}$-i [5] | i-$H_{19}C_9NHCSNH_2$ [6] 0,1 | 0,22 [3] | 0,25 [3] | 24 | 74,6 %<br>170°C |
| 14 [8] | Oleyl | Oleyl-$NHCSNH_2$ 0,032 | 0,0704 [3] | 0,08 [3] | 24 | 93 %<br>97°C (nach Umkristallisation aus Methanol) |

| Bsp. | $R_2$ | Reaktanden | | | Reaktionszeit in Stunden | Ausbeute / Schmelzpunkt |
|---|---|---|---|---|---|---|
| | | Thioharnstoff (Mol) | Methylacetoacetat (Mol) | NaOMe (Mol) | | |
| 15 [9] | $-C_{18}H_{37}$-n | n-$H_{37}C_{18}NHCSNH_2$ 0,6 | 1,4 [2] | 1,4 [2] | 16 | <br>130°C |
| 16 [10] | $-C_4H_9$-i | i-$H_9C_4NHCSNH_2$ [6] 0,3 | 0,66 [3] | 0,75 [3] | 24 | <br>200°C |

Indices:

1) $-C_8H_{17}$-i entspricht

$$-CH_2-\underset{C_2H_5}{CH}-(CH_2)_3-CH_3.$$

2) Die Zugabe erfolgt in zwei Portionen. Die erste Hälfte wird zu Beginn der Umsetzung zugegeben und die zweite Hälfte nach 4 Stunden Reaktionszeit.
3) Die Zugabe erfolgt in zwei Portionen. Die erste Hälfte wird zu Beginn der Umsetzung zugegeben und die zweite Hälfte nach 8 Stunden Reaktionszeit.
4) Der Rückstand wird in 250 ml Wasser und 100 ml einer 15%igen wässrigen Natriumhydroxidlösung aufgenommen. Der erhaltene Niederschlag wird abfiltriert (Rückisolierung von 13 % des nicht umgesetzten Thioharnstoffs). Das Filtrat wird angesäuert und gemäss Beispiel 9 aufgearbeitet.
5) $-C_9H_{19}$-i entspricht

$$-CH_2-CH_2-\underset{CH_3}{CH}-CH_2-\overset{CH_3}{\underset{CH_3}{C}}-CH_3.$$

6) Die Reinheit des verwendeten Thioharnstoffs beträgt ca. 80 %.
7) Der Rückstand wird in 250 ml Wasser und 100 ml einer 15%igen wässrigen Natriumhydroxidlösung aufgenommen, wobei dieser in Lösung geht. Die Lösung wird angesäuert und gemäss Beispiel 9 aufgearbeitet.
8) Der Rückstand wird in 300 ml Wasser und 300 ml Essigester aufgenommen und mit konz. HCl angesäuert. Die organische Phase wird abgetrennt, mit Wasser gewaschen, getrocknet und eingeengt.
9) Der Rückstand wird in 1700 ml einer 20%igen wässrigen Natriumhydroxidlösung 15 Minuten erhitzt und auf 2,5 kg Eis und 1500 ml Aceton gegeben. Der erhaltene Niederschlag wird abfiltriert und in 3600 ml Ethanol gelöst. Durch fraktionierte Fällung lassen sich 44,3 % des eingesetzten Thioharnstoffs zurückgewinnen. Aus den vereinigten Filtraten wird durch Ansäuern das 2-Thiouracil erhalten.
10) Der Rückstand wird in 300 ml Wasser und 50 ml Methylenchlorid aufgenommen und gemäss Beispiel 9 aufgearbeitet.

Beispiel 17: Herstellung von 3-Methyl-6-phenyl-2-thiouracil.

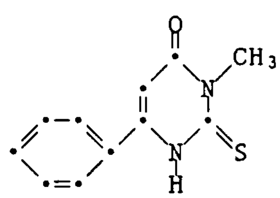

Die Herstellung erfolgt in Analogie zu Beispiel 9. Es werden 0,3 Mol N-Methylthioharnstoff, 0,66 Mol Ethylbenzoylacetat und 0,75 Mol Natriummethylat in Form einer 30%igen methanolischen Natriummethylatlösung eingesetzt. Ethyl-benzoylacetat und Natriummethylat werden dem Reaktionsgemisch in 2 Portionen zugegeben. Die Zugabe der ersten Hälfte erfolgt zu Beginn der Umsetzung und die zweite Hälfte wird nach 10 Stunden Reaktionszeit hinzugefügt. Die Reaktionszeit beträgt 24 Stunden. Nach Beendigung der Umsetzung wird der Rückstand in 100 ml Isopropylether und 300 ml Wasser gelöst und mit konzentrierter Salzsäure angesäuert. Der erhaltene Niederschlag wird gemäss Beispiel 9 aufgearbeitet. Das Produkt besitzt einen Schmelzpunkt von 236 ° C.

Beispiel 18: Herstellung von 3-(2'-Methoxyethyl)-6-methyl-2-thiouracil.

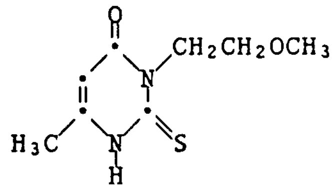

47,0 g (0,35 Mol) N-(2-Methoxyethyl)thioharnstoff werden in 150 ml abs. Methanol gelöst. Unter Rühren werden 69,7 g (0,60 Mol) Methylacetoacetat und 126 g (0,70 Mol) einer 30%igen methanolischen Natriummethylatlösung zugegeben. Das Reaktionsgemisch wird 19 Stunden am Rückfluss erhitzt. Anschliessend wird Methanol abdestilliert und der erhaltene Rückstand in 200 ml Wasser gelöst und mit konz. Salzsäure angesäuert. Der entstandene Niederschlag wird abgesaugt, neutral gewaschen und bis zur Gewichtskonstanz getrocknet. Das Produkt besitzt einen Schmelzpunkt von 200 °C. Die Ausbeute beträgt 21,3 g (≙ 30,4 % der Theorie).

Beispiel 19: Herstellung von 1-Benzoyl-3,6-dimethyl-2-thiouracil.

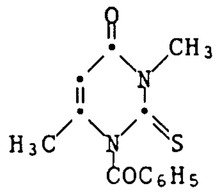

In einem 1-l-Dreihalskolben werden 53,5 g (0,34 Mol) 3,6-Dimethyl-2-thiouracil und 61,7 g (0,34 Mol) einer 30%igen Natriummethylatlösung in 400 ml abs. Methanol unter Rühren 60 min am Rückfluss erhitzt. Anschliessend werden von der klaren Lösung ca. 400 ml Methanol abdestilliert und unter Erhitzen sukzessive Toluol zugegeben, bis bei der azeotropen Destillation der Siedepunkt des Toluols erreicht ist. Es resultiert eine heterogene Suspension eines methanolfreien Thiouracilnatriumsalzes. Hierauf wird das Toluol grösstenteils abgezogen und durch 300 ml Dimethylacetamid ersetzt, wobei eine homogene Lösung eines methanolfreien Thiouracilnatriumsalzes erhalten wird. Bei 20 °C werden nun 62,2 g (0,44 Mol) Benzoylchlorid zugetropft und das Reaktionsgemisch 60 min gerührt. Das ausgefallene Kochsalz wird abfiltriert und das Filtrat im Vakuum von den flüchtigen Bestandteilen befreit. Der Rückstand wird mit 300 ml Isopropylether*)-behandelt. Der Niederschlag wird abfiltriert. Nach Trocknung resultieren 77,6 g eines Isomerengemisches aus 1-Benzoyl-3,6-dimethyl-2-thiouracil und 2-Benzoylthio-3,6-dimethyl-4-oxo-3,4-dihydropyrimidin. Das Gemisch wird aus abs. Aceton umkristallisiert. Man erhält farblose Kristalle, die bei 163 °C schmelzen. Gemäss $^{1}$H-NMR-Spektrum besteht das Gemisch zu 90 % aus 1-Benzoyl-3,6-dimethyl-2-thiouracil.

Das Isomerengemisch wird mit einer wässrigen Bicarbonatlösung behandelt, wobei das Dihydropyrimidin hydrolytisch zersetzt wird. 1-Benzoyl-3,6-dimethyl-2-thiouracil, welches einen Schmelzpunkt von 173 °C besitzt, lässt sich auf diese Weise nahezu quantitativ abtrennen.

Beispiel 20: Herstellung von 1,3-Dibenzoyl-6-methyl-2-thiouracil.

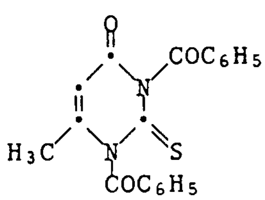

*)Die Aufarbeitung kann auch mit Bicarbonatlösung anstelle von Isopropylether durchgeführt werden. In diesem Fall erhält man das gewünschte Thiouracil direkt unter Verlust des Dihydropyrimidins.

In Analogie Zu Beispiel 19 wird ein Thiouracil-Dinatriumsalz, welches heterogen in Toluol vorliegt, aus 85,3 g (0,6 Mol) 6-Methyl-2-thiouracil, 216 g (1,2 Mol) einer 30%igen Natriummethylatlösung in 500 ml abs. Methanol und Toluol hergestellt. Die Suspension wird 3h am Rückfluss mit 168,7 g (1,2 Mol) Benzoylchlorid umgesetzt. Das erhaltene Kochsalz und nichtumgesetztes Thiouracil-Dinatriumsalz werden abfiltriert. Das Filtrat wird im Vakuum von flüchtigen Bestandteilen befreit und der Rückstand (115,3 g) aus 600 ml Isopropanol/Methylenchlorid (5:1) umkristallisiert. Das erhaltene 1,3-Dibenzoyl-6-methyl-2-thiouracil besitzt einen Schmelzpunkt von 76 ° C. Die Ausbeute beträgt 92,1 g (≙ 62,3 % der Theorie).

Beispiele 21-24: Die in der Tabelle 3 aufgeführten Verbindungen werden in Analogie zu Beispiel 20 hergestellt.

Tabelle 3:

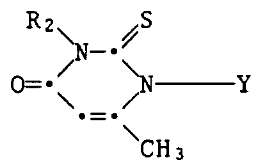

| Beispiel | $R_2$ | Y | Ausbeute / Schmelzpunkt |
|---|---|---|---|
| 21 | $-CH_3$ | $-\underset{\underset{O}{\|}}{C}-C_{11}H_{23}-n$ | 42 % / 116°C (Nach Umkristallisation aus Aceton) |
| 22 | $-CH_3$ | $-\underset{\underset{O}{\|}}{C}O-C_2H_5$ | 64,4 % / 98°C (Nach Umkristallisation aus Isopropylether) |
| 23 | $-CH_2-CH=CH_2$ | $-\underset{\underset{O}{\|}}{C}-C_6H_5$ | 33,4 % / 95°C (Nach Umkristallisation aus Methylenchlorid/Petrolether) |
| 24[11)] | $-CH_2C_6H_5$ | $-\underset{\underset{O}{\|}}{C}-C_6H_5$ | 35,2 % / 163°C (Nach Umkristallisation aus Methylenchlorid/Isopropylether) |

[11)] Liegt als Gemisch aus 1-Benzoyl-3-benzyl-6-methyl-2-thiouracil und 2-Benzoylthio-3-benzyl-6-methyl-4-oxo-3,4-dihydropyrimidin vor.

Beispiel 25: Herstellung von 1-i-Nonanoyl-3,6-dimethyl-2-thiouracil.

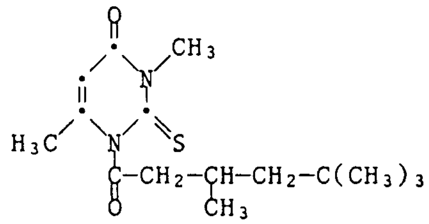

31,2 g (0,2 Mol) 3,6-Dimethyl-2-thiouracil und 36,0 g (0,2 Mol) einer 30%igen methanolischen Natriummethylatlösung werden in 200 ml abs. Methanol 2h am Rückfluss erhitzt. In der resultierenden klaren Lösung wird das enthaltene Methanol durch Toluol mit Hilfe des Azeotropverfahrens ersetzt. Die Natriumsalzsuspension wird durch Abdestillation von Toluol aufkonzentriert. Durch Zugabe von 150 ml abs. Dimethylacetamid erhält man eine klare Lösung. Nach Zugabe von 35,3 g (0,2 Mol) Isononansäurechlorid wird das Reaktionsgemisch über Nacht bei Raumtemperatur gerührt. Die Lösung wird in 600 ml Eiswasser eingerührt, der Niederschlag salzfrei gewaschen und bis zur Gewichtskonstanz getrocknet. Das erhaltene Produkt ist ein 1:1 Gemisch aus 1-i-Nonanoyl-3,6-dimethyl-2-thiouracil und 2-i-Nonanoylthio-3,6-dimethyl-4-oxo-3,4-dihydropyrimidin. Die Ausbeute beträgt 56,6 g (≙ 95,4 % der Theorie).

Das Isomerengemisch, welches chromatographisch trennbar ist, besitzt einen Schmelzpunkt von 129 °C.

Beispiel 26: Herstellung von 1-(2′,4′-Dichlorbenzoyl)-3,6-dimethyl-2-thiouracil.

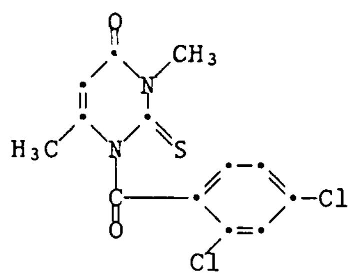

Die Herstellung erfolgt in Analogie zu Beispiel 25. Als Acylierungsreagenz wird 2,4-Dichlorbenzoylchlorid verwendet. Es werden 28,9 g (≙ 43,9 % der Theorie) eines 1,5:1 Gemisches aus 1-(2′,4′-Dichlorbenzoyl)-3,6-dimethyl-2-thiouracil und 2-(2′,4′-Dichlorbenzoylthio)-3,6-dimethyl-4-oxo-3,4-dihydropyrimidin erhalten. Der Schmelzpunkt des Isomerengemisches beträgt 160 °C. Reines 1-(2′,4′-Dichlorbenzoyl)-3,6-dimethyl-2-thiouracil kann aus dem Isomerengemisch durch Zersetzung des Dihydropyrimidins mit Hilfe einer NaOH Lösung erhalten werden.

Beispiel 27: Herstellung von 1-(2′-Ethylhexyloxycarbonyl)-3,6-dimethyl-2-thiouracil.

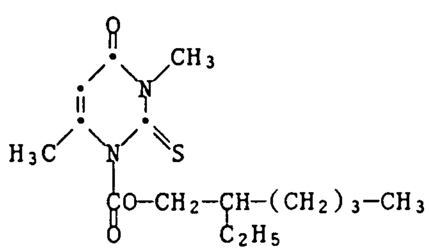

Die Herstellung erfolgt in Analogie zu Beispiel 25. Als Acylierungsreagenz wird Isooctylchlorformiat verwendet. Nach Zugabe von Dimethylacetamid, Acylierungsreagenz und Rühren bei Raumtemperatur werden im Vakuum die flüchtigen Bestandteile abgezogen. Der Rückstand wird mit Petrolether ausgekocht und vom gebildeten Kochsalzniederschlag abfiltriert. Das Filtrat wird bis zum Rückstand eingeengt. Das erhaltene 1-(2′Ethylhexyloxycarbonyl)-3,6-dimethyl-2-thiouracil liegt als viskoses hellbraunes Oel vor und besitzt einen Brechungsindex von $n_D^{20}$ = 1,5360.

Beispiel 28: Herstellung von 1-(n-Octylthio)carbonyl-3,6-dimethyl-2-thiouracil.

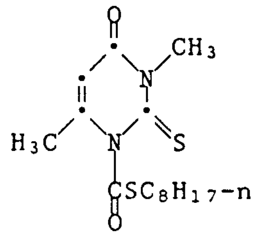

Die Herstellung erfolgt in Analogie zu Beispiel 25. Als Acylierungsreagenz wird n-Octylthiochlorformiat verwendet. Nach der Umsetzung wird die Dimethylacetamidlösung in Eiswasser eingerührt, wobei mehrfach mit Methylenchlorid extrahiert wird. Die organische Phase wird mit Wasser ausgeschüttelt, getrocknet und anschliessend bis zum Rückstand eingeengt. Der Rückstand wird mit Petrolether behandelt und filtriert. Das Filtrat wird von den flüchtigen Bestandteilen im Vakuum entfernt. Es wird ein 4:1 Gemisch aus 1-(n-Octylthio)carbonyl-3,6-dimethyl-2-thiouracil und 2-(n-Octylthio)carbonylthio-3,6-dimethyl-4-oxo-3,4-dihydropyrimidinerhalten. Das Isomerengemisch liegt als farbloses Wachs vor und besitzt einen Schmelzpunkt von 100 ° C.

Beispiel 29: Herstellung von 1-Diethylcarbamoyl-3,6-dimethyl-2-thiouracil.

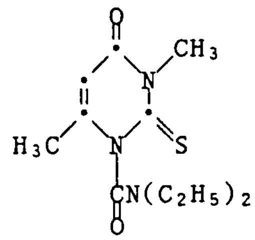

Die Herstellung erfolgt in Analogie zu Beispiel 25. Als Acylierungsreagenz wird Diethylcarbamidsäurechlorid verwendet. Nach Zugabe von Dimethylacetamid, Acylierungsreagenz und Rühren bei Raumtemperatur wird das Reaktionsgemisch bis zum Rückstand eingeengt. Dieser wird mit Ether ausgekocht, filtriert und das Filtrat erneut eingeengt. Der so erhaltene Rückstand wird mit Petrolether ausgekocht und der entstandene Niederschlag abfiltriert. 1-Diethylcarbamoyl-3,6-dimethyl-2-thiouracil wird als farbloser Feststoff erhalten, der einen Schmelzpunkt von 90 ° C besitzt.

Beispiel 30: Herstellung von 1-Dimethylthiocarbamoyl-3,6-dimethyl-2-thiouracil.

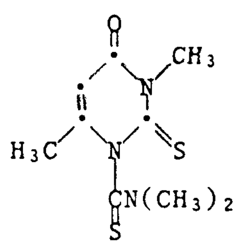

Die Herstellung erfolgt in Analogie zu Beispiel 25. Als Acylierungsreagenz wird Dimethylthiocarbamidsäurechlorid verwendet. Nach der Umsetzung wird die Dimethylacetamidlösung in Eiswasser eingerührt, der erhaltene Niederschlag abfiltriert und das Filtrat mit Methylenchlorid ausgeschüttelt. Die organische Phase wird getrocknet und bis zum Rückstand eingeengt, welcher mit Ether behandelt wird. Es scheidet sich ein Feststoff ab, der mit dem ursprünglichen Niederschlag vereint wird. Das so erhaltene Produkt wird nochmals mit Ether behandelt. 1-Dimethylthiocarbamoyl-3,6-dimethyl-2-thiouracil liegt als farbloser Feststoff vor, welcher einen Schmelzpunkt von 149 ° C besitzt.

Beispiel 31:

Eine Trockenmischung bestehend aus 100 Teilen S-PVC (K-Wert 64), 3 Teilen epoxidiertem Sojabohnenöl, 0,35 Teilen Ca-Stearat, 0,15 Teilen Zn-Stearat, 0,55 Teilen Diisodecyl-phenylphosphit und 0,3 Teilen des in den Tabellen 4a bis 4d angegebenen Stabilisators wird auf einem Mischwalzwerk 5 Minuten bei 180 °C gewalzt. Vom gebildeten 0,3 mm dicken Walzfell werden Folienmuster in einem Testofen (®Mathis Thermotester Typ LTF-ST) bei 180 °C thermisch belastet. Im unten angegebenen Zeitintervall wird an einem Prüfmuster der "Yellowness Index" (YI) nach ASTM D 1925 bestimmt. Die Ergebnisse sind in den Tabellen 4a bis 4d zusammengefasst.

Tabelle 4a

| Stabilisator | YI nach Belastungszeit in Minuten | | | | |
|---|---|---|---|---|---|
| | 0 | 5 | 10 | 15 | 20 |
| ohne | 19,1 | 20,1 | 24,6 | 32,4 | 37,3 |
| Verbindung aus Beispiel 5 | 1,9 | 2,1 | 2,7 | 4,5 | 15,5 |
| Verbindung aus Beispiel 6 | 2,0 | 2,4 | 2,8 | 3,9 | 14,4 |
| Verbindung aus Beispiel 9 | 2,1 | 2,6 | 3,2 | 5,5 | 14,6 |

Tabelle 4b

| Stabilisator | YI nach Belastungszeit in Minuten | | | |
|---|---|---|---|---|
| | 0 | 5 | 10 | 15 |
| ohne | 22,2 | 35,5 | 39,2 | 39,4 |
| Verbindung aus Beispiel | 25 5,1 | 5,7 | 7,0 | 11,4 |

Tabelle 4c

| Stabilisator | YI nach Belastungszeit in Minuten | | | |
|---|---|---|---|---|
| | 0 | 5 | 10 | 15 |
| ohne | 17,9 | 40,7 | 42,1 | 40,5 |
| Verbindung aus Beispiel | 8 2,0 | 4,3 | 8,1 | 15,7 |
| Verbindung aus Beispiel | 13 2,3 | 3,4 | 5,1 | 15,1 |

Tabelle 4d

| Stabilisator | YI nach Belastungszeit in Minuten | | | |
|---|---|---|---|---|
| | 0 | 5 | 10 | 15 |
| ohne | 17,6 | 37,6 | 40,2 | 41,0 |
| Verbindung aus Beispiel 7 | 1,8 | 3,7 | 4,5 | 10,4 |
| Verbindung aus Beispiel 10 | 2,0 | 3,3 | 4,4 | 8,6 |
| Verbindung aus Beispiel 11 | 3,4 | 4,5 | 5,8 | 9,5 |
| Verbindung aus Beispiel 12 | 1,9 | 3,2 | 5,4 | 14,7 |

Beispiel 32: Eine Trockenmischung bestehend aus 100 Teilen S-PVC (K-Wert 70), 17 Teilen Dioctylphthalat, 3 Teilen epoxidiertem Sojabohnenöl, 0,33 Teilen Zn-Oleat, 0,53 Teilen Ba-p-(t-butyl)benzoat, 0,7 Teilen Diisodecyl-phenylphosphit, 0,44 Teilen ®SHELL SOL A (aromatisches Kohlenwasserstoff-Gemisch) und 0,2 Teilen des in den Tabellen 5a bis 5e angegebenen Stabilisators wird auf einem Mischwalzwerk 5 Minuten bei 190°C gewalzt. Vom gebildeten 0,3 mm dicken Walzfell werden Folienmuster in einem Testofen (®Mathis-Thermotester) bei 180°C thermisch belastet. Im angegebenen Zeitintervall wird an einem Prüfmuster der "Yellowness Index" (YI) nach ASTM D 1925 bestimmt. Die Ergebnisse sind in den Tabellen 5a bis 5e zusammengefasst.

Tabelle 5a

| Stabilisator | YI nach Belastungszeit in Minuten | | | | | | | | | |
|---|---|---|---|---|---|---|---|---|---|---|
| | 0 | 5 | 10 | 15 | 20 | 25 | 30 | 35 | 40 | 45 |
| ohne | 10,5 | 11,6 | 13,3 | 19,8 | 22,2 | 26,4 | 27,7 | 26,6 | 24,0 | 20,0 |
| Verbindung aus Beispiel 5 | 0,4 | 0,6 | 0,9 | 1,2 | 1,4 | 2,2 | 4,6 | 5,4 | 8,6 | 13,3 |
| Verbindung aus Beispiel 6 | 0,1 | 0,6 | 0,9 | 0,9 | 1,5 | 1,9 | 2,7 | 4,2 | 7,0 | 9,6 |
| Verbindung aus Beispiel 9 | 0,3 | 0,3 | 0,5 | 0,4 | 1,1 | 1,3 | 2,6 | 4,0 | 6,1 | 8,7 |

Tabelle 5b

| Stabilisator | YI nach Belastungszeit in Minuten | | | | |
|---|---|---|---|---|---|
| | 0 | 5 | 10 | 15 | 30 |
| ohne | 9,2 | 11,5 | 13,2 | 17,1 | 22,4 |
| Verbindung aus Beispiel 1 | 1,4 | 1,9 | 2,3 | 2,6 | 3,1 |
| Verbindung aus Beispiel 22 | 1,9 | 2,1 | 2,5 | 2,7 | 3,2 |
| Verbindung aus Beispiel 25 | 2,9 | 3,4 | 3,5 | 4,0 | 5,2 |
| Verbindung aus Beispiel 27 | 2,1 | 2,8 | 3,0 | 3,2 | 4,1 |
| Verbindung aus Beispiel 28 | 1,7 | 2,4 | 2,7 | 3,7 | 5,7 |

Tabelle 5c

| Stabilisator | YI nach Belastungszeit in Minuten | | | | |
|---|---|---|---|---|---|
| | 0 | 5 | 10 | 15 | 30 |
| ohne | 9,8 | 20,4 | 24,7 | 25,8 | 26,6 |
| Verbindung aus Beispiel 19 | 1,6 | 2,2 | 2,8 | 2,9 | 5,2 |
| Verbindung aus Beispiel 23 | 1,0 | 1,7 | 2,0 | 2,6 | 3,5 |
| Verbindung aus Beispiel 24 | 1,7 | 2,1 | 2,9 | 3,1 | 3,4 |

Tabelle 5d

| Stabilisator | YI nach Belastungszeit in Minuten | | | | |
|---|---|---|---|---|---|
| | 0 | 5 | 10 | 15 | 30 |
| ohne | 8,1 | 17,0 | 20,2 | 23,0 | 23,4 |
| Verbindung aus Beispiel 4 | 1,3 | 2,8 | 2,7 | 3,0 | 4,0 |
| Verbindung aus Beispiel 8 | 1,0 | 2,4 | 2,7 | 3,6 | 5,0 |
| Verbindung aus Beispiel 13 | 1,9 | 2,2 | 2,6 | 3,4 | 4,2 |
| Verbindung aus Beispiel 21 | 1,9 | 3,0 | 3,2 | 4,4 | 6,3 |

Tabelle 5e

| Stabilisator | YI nach Belastungszeit in Minuten | | | | |
|---|---|---|---|---|---|
| | 0 | 5 | 10 | 15 | 20 |
| ohne | 10,6 | 13,0 | 16,3 | 19,7 | 22,8 |
| Verbindung aus Beispiel 7 | 1,4 | 1,6 | 2,2 | 2,5 | 3,0 |
| Verbindung aus Beispiel 10 | 1,4 | 2,1 | 2,5 | 3,1 | 4,5 |
| Verbindung aus Beispiel 11 | 1,8 | 3,0 | 3,1 | 3,1 | 4,3 |
| Verbindung aus Beispiel 12 | 1,7 | 2,1 | 2,6 | 2,8 | 4,0 |

Beispiel 33: Eine Trockenmischung bestehend aus 100 Teilen S-PVC (K-Wert 70), 17 Teilen Dioctylphthalat, 3 Teilen epoxidiertem Sojabohnenöl, 0,26 Teilen ®SHELL SOL A (aromatisches Kohlenwasserstoff-Gemisch), 0,48 Teilen Zn-Oleat, 0,54 Teilen Ba-p-(t-butyl)benzoat, 0,64 Teilen Diisodecyl-phenylphosphit, 0,06 Teilen 2,6-Di-t-butyl-4-methylphenol, 0,02 Teilen Oelsäure und 0,2 Teilen des in Tabelle 6 angegebenen Stabilisators wird auf einem Mischwalzwerk 5 Minuten bei 190 °C gewalzt. Vom gebildeten 0,3 mm dicken Walzfell werden Folienmuster in einem Trockenschrank bei 180 °C thermisch belastet. Der Yellowness Index (YI) der Proben wird in regelmässigen Zeitabständen nach ASTM D 1925 bestimmt. Die Ergebnisse sind in Tabelle 6 aufgeführt.

Tabelle 6

| Stabilisator | YI nach Belastungszeit in Minuten | | | | | | |
|---|---|---|---|---|---|---|---|
| | 0 | 10 | 20 | 30 | 40 | 50 | 60 |
| Verbindung aus Beispiel 1 | 2,8 | 3,9 | 4,3 | 5,5 | 6,2 | 9,0 | 12,8 |

Beispiel 34: Eine Trockenmischung bestehend aus 100 Teilen S-PVC (K-Wert 64), 3 Teilen epoxidiertem Sojabohnenöl, 0,35 Teilen Ca-Stearat, 0,15 Teilen Zn-Stearat, 0,55 Teilen Diisodecyl-phenylphosphit und 0,3 Teilen des in Tabelle 7 angegebenen Stabilisators wird auf einem Mischwalzwerk 5 Minuten bei 180 °C gewalzt. Vom gebildeten 0,3 mm dicken Walzfell werden Folienmuster in einem ®VITALUX-Gerät von ARA belichtet.

Folgende Prüfbedingungen liegen vor:

Ohne Beregnung
Strahlungsquelle: 8 Osram-Vitalux Lampen zu je 300 Watt
Normaltemperatur: 46 °C
Schwarztafeltemperatur: 65 °C

Keine Filtereinstellung

Tabelle 7

| Stabilisator | YI nach Belastungszeit in Minuten | | | |
|---|---|---|---|---|
| | 0 | 5 | 10 | 15 |
| ohne | 14,8 | 30,6 | 35,9 | 36,8 |
| Verbindung aus Beispiel 1 | 1,6 | 3,1 | 5,1 | 16,6 |
| Verbindung aus Beispiel 8 | 1,5 | 3,1 | 5,3 | 13,8 |

## Patentansprüche

1. Zusammensetzung enthaltend a) ein chlorhaltiges Polymerisat und b) mindestens eine Verbindung der Formel I,

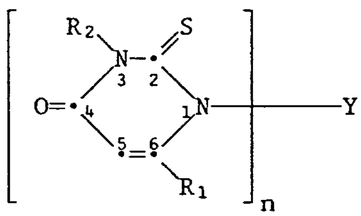

(I)

worin n 1 oder 2 bedeutet, $R_1$ $C_1$-$C_{18}$-Alkyl, $C_3$-$C_{18}$-Alkenyl, Phenyl, $C_7$-$C_{12}$-Phenylalkyl oder am Phenylring durch $C_1$-$C_4$-Alkyl und/oder Chlor substituiertes $C_7$-$C_{12}$-Phenylalkyl ist, $R_2$ $C_1$-$C_{18}$-Alkyl, eine Gruppe

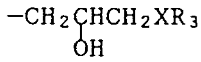

oder durch Hydroxy oder eine Gruppe $-XR_3$ substituiertes $C_2$-$C_{22}$-Alkyl ist, wobei X Sauerstoff oder Schwefel ist und $R_3$ $C_1$-$C_{18}$-Alkyl, Phenyl oder $C_7$-$C_{12}$-Phenylalkyl darstellt, oder $R_2$ ferner $C_3$-$C_{18}$-Alkenyl,$C_7$-$C_{12}$-Phenylalkyl, am Phenylring durch $C_1$-$C_4$-Alkyl und/oder Chlor substituiertes $C_7$-$C_{12}$-Phenylalkyl, Di($C_1$-$C_4$-alkyl)thiocarbamoyl oder eine Gruppe der Formel IIa oder IIb ist,

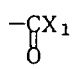 , 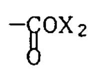

(IIa) (IIb)

wobei $X_1$ $C_1$-$C_{17}$-Alkyl, $C_3$-$C_{17}$-Alkenyl, $C_5$-$C_7$-Cycloalkyl, durch $C_1$-$C_4$-Alkyl substituiertes $C_5$-$C_7$-Cycloalkyl, Phenyl, durch $C_1$-$C_4$-Alkyl und/oder Chlor substituiertes Phenyl, $C_7$-$C_{12}$-Phenylalkyl, am Phenylring durch $C_1$-$C_4$-Alkyl und/oder Chlor substituiertes $C_7$-$C_{12}$-Phenylalkyl, $C_1$-$C_{10}$-Alkylthio oder Di($C_1$-$C_4$-alkyl)amino bedeutet und $X_2$ $C_1$-$C_{18}$-Alkyl, $C_3$-$C_{18}$-Alkenyl, $C_5$-$C_7$-Cycloalkyl, durch $C_1$-$C_4$-Alkyl substituiertes $C_5$-$C_7$-Cycloalkyl, Phenyl, durch $C_1$-$C_4$-Alkyl und/oder Chlor substituiertes Phenyl, $C_7$-$C_{12}$-Phenylalkyl oder am Phenylring durch $C_1$-$C_4$-Alkyl und/oder Chlor substituiertes $C_7$-$C_{12}$-Phenylalkyl ist, wenn n 1 bedeutet, Y Di($C_1$-$C_4$-alkyl)thiocarbamoyl oder eine Gruppe der Formel IIa oder IIb ist, wenn n 2 bedeutet, Y eine Gruppe IIc oder IId ist,

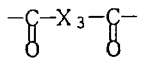 , 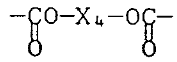

(IIc) (IId)

wobei $X_3$ $C_1$-$C_{12}$-Alkylen oder Phenylen bedeutet und $X_4$ $C_2$-$C_{12}$-Alkylen oder durch 1 oder 2 Sauerstoffatome unterbrochenes $C_4$-$C_8$-Alkylen ist, mit der Massgabe, dass die Reste Y und $R_2$ gleich sind, wenn $R_2$ Di($C_1$-$C_4$-alkyl)-thiocarbamoyl oder eine Gruppe der Formel IIa oder IIb bedeutet.

**2.** Zusammensetzung gemäss Anspruch 1, worin n 1 ist.

**3.** Zusammensetzung gemäss Anspruch 1, worin n 1 ist, $R_1$ $C_1$-$C_4$-Alkyl oder Phenyl bedeutet, $R_2$ $C_1$-$C_{18}$-Alkyl, durch Hydroxy oder $C_1$-$C_6$-Alkyloxy substituiertes $C_2$-$C_6$-Alkyl, $C_3$-$C_{18}$-Alkenyl, Benzyl oder Benzoyl ist, Y Di($C_1$-$C_4$-alkyl)thiocarbamoyl oder eine Gruppe IIa oder IIb ist, $X_1$ $C_1$-$C_{12}$-Alkyl, Phenyl, Dichlorphenyl, $C_1$-$C_{10}$-Alkylthio oder Di($C_1$-$C_4$-alkyl)amino bedeutet und $X_2$ $C_1$-$C_{12}$-Alkyl darstellt.

**4.** Zusammensetzung gemäss Anspruch 1, worin m 1 ist, $R_1$ Methyl bedeutet, $R_2$ $C_1$-$C_4$-Alkyl, 2-Hydroxyethyl, 2-Methoxyethyl oder Allyl ist und Y Benzoyl oder ($C_1$-$C_{12}$-Alkyl)oxycarbonyl bedeutet.

**5.** Zusammensetzung gemäss Anspruch 1, worin $R_1$ $C_1$-$C_4$-Alkyl oder Phenyl bedeutet.

**6.** Zusammensetzung gemäss Anspruch 1, worin $R_1$ Methyl bedeutet.

**7.** Zusammensetzung gemäss Anspruch 1, worin $R_2$ $C_1$-$C_{18}$-Alkyl, durch Hydroxy oder $C_1$-$C_6$-Alkyloxy substituiertes $C_2$-$C_6$-Alkyl, $C_3$-$C_{18}$-Alkenyl, Benzyl oder Benzoyl bedeutet.

**8.** Zusammensetzung enthaltend a) ein chlorhaltiges Polymerisat und b) mindestens eine Verbindung der Formel I-A,

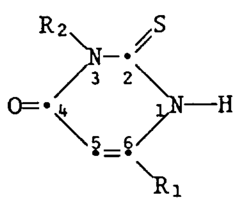

(I-A)

worin $R_1$ $C_1$-$C_4$-Alkyl oder Phenyl bedeutet und $R_2$ $C_1$-$C_{18}$-Alkyl, durch Hydroxy oder $C_1$-$C_6$-Alkyloxy substituiertes $C_2$-$C_6$-Alkyl, $C_3$-$C_{18}$-Alkenyl, Benzyl oder Benzoyl ist, und als zusätzliche Komponente c) eine Epoxyverbindung.

**9.** Zusammensetzung gemäss Anspruch 8, worin die Komponente b)
3-Methyl-6-n-propyl-2-thiouracil,
3-Methyl-6-t-butyl-2-thiouracil,
3-Ethyl-6-methyl-2-thiouracil,
3-Allyl-6-methyl-2-thiouracil,
3-Benzyl-6-methyl-2-thiouracil,
3-n-Octyl-6-methyl-2-thiouracil,
3-(2'-Hydroxyethyl)-6-methyl-2-thiouracil,
3-n-Butyl-6-methyl-2-thiouracil,
3-i-Octyl-6-methyl-2-thiouracil
3-n-Decyl-6-methyl-2-thiouracil,
3-n-Dodecyl-6-methyl-2-thiouracil,
3-i-Nonyl-6-methyl-2-thiouracil,
3-Oleyl-6-methyl-2-thiouracil,
3-n-Octadecyl-6-methyl-2-thiouracil,
3-i-Butyl-6-methyl-2-thiouracil,
3-Methyl-6-phenyl-2-thiouracil oder

3-(2'-Methoxyethyl)-6-methyl-2-thiouracil ist.

**10.** Zusammensetzung gemäss Anspruch 1 oder 8, enthaltend zusätzlich mindestens ein Me(II)-Carboxylat und/oder Me(II)-Phenolat, wobei Me(II) Ba, Ca, Mg, Cd oder Zn bedeutet.

**11.** Zusammensetzung gemäss Anspruch 1 oder 8, enthaltend zusätzlich mindestens ein Me(II)-Carboxylat, wobei Me(II) Ba, Ca, Mg oder Zn bedeutet.

**12.** Zusammensetzung gemäss Anspruch 1, enthaltend als zusätzliche Komponente c) eine Epoxyverbindung.

**13.** Zusammensetzung gemäss Anspruch 12, enthaltend zusätzlich mindestens ein Me(II)-Carboxylat und/oder Me(II)-Phenolat, wobei Me(II) Ba, Ca, Mg, Cd oder Zn bedeutet.

**14.** Zusammensetzung gemäss Anspruch 8, worin Komponente b) 3,6-Dimethyl-2-thiouracil ist.

**15.** Verbindungen der im Anspruch 1 definierten Formel I.

**16.** Verbindungen gemäss Anspruch 15, worin n 1 bedeutet, $R_1$ $C_1$-$C_4$-Alkyl ist, $R_2$ $C_1$-$C_4$-Alkyl, Allyl, Benzyl oder Benzoyl bedeutet und Y Di($C_1$-$C_4$-alkyl)thiocarbamoyl oder eine Gruppe IIa oder IIb ist, $X_1$ $C_1$-$C_{12}$-Alkyl, Phenyl, Dichlorphenyl, $C_1$-$C_{10}$-Alkylthio oder Di($C_1$-$C_4$-alkyl)amino bedeutet und $X_2$ $C_1$-$C_{12}$-Alkyl darstellt.

**17.** Die Verbindungen
3-Methyl-6-n-propyl-2-thiouracil,
3-Methyl-6-t-butyl-2-thiouracil,
3-(2'-Hydroxyethyl)-6-methyl-2-thiouracil,
3-n-Decyl-6-methyl-2-thiouracil,
3-n-Dodecyl-6-methyl-2-thiouracil,
3-Oleyl-6-methyl-2-thiouracil,
3-n-Octadecyl-6-methyl-2-thiouracil,
3-Methyl-6-phenyl-2-thiouracil und
3-(2'-Methoxyethyl)-6-methyl-2-thiouracil.

## Claims

**1.** A composition containing a) a chlorine-containing polymer and b) at least one compound of the formula I

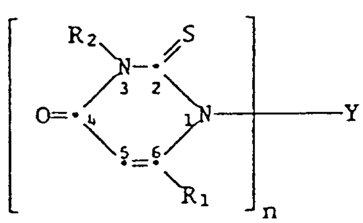

(I)

in which n is 1 or 2, $R_1$ is $C_1$-$C_{18}$alkyl, $C_3$-$C_{18}$alkenyl, phenyl, $C_7$-$C_{12}$phenylalkyl or $C_7$-$C_{12}$phenylalkyl which is substituted on the phenyl ring by $C_1$-$C_4$alkyl and/or chlorine, $R_2$ is $C_1$-$C_{18}$alkyl, a group

$$-CH_2CHCH_2XR_3$$
$$OH$$

or $C_2$-$C_{22}$alkyl which is substituted by hydroxyl or a group $-XR_3$, in which X is oxygen or sulfur and $R_3$ is $C_1$-$C_{18}$alkyl, phenyl or $C_7$-$C_{12}$phenylalkyl, or $R_2$ is furthermore $C_3$-$C_{18}$alkenyl, $C_7$-$C_{12}$phenylalkyl, $C_7$-$C_{12}$phenylalkyl which is substituted on the phenyl ring by $C_1$-$C_4$alkyl and/or chlorine, di($C_1$-$C_4$alkyl)-

thiocarbamoyl or a group of the formula IIa or IIb

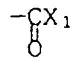 , 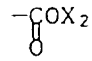

(IIa) (IIb)

in which $X_1$ is $C_1$-$C_{17}$alkyl, $C_3$-$C_{17}$alkenyl, $C_5$-$C_7$cycloalkyl, $C_1$-$C_4$alkyl-substituted $C_5$-$C_7$cycloalkyl, phenyl, $C_1$-$C_4$alkyl- and/or chlorine-substituted phenyl, $C_7$-$C_{12}$phenylalkyl, $C_7$-$C_{12}$phenylalkyl which is substituted on the phenyl ring by $C_1$-$C_4$alkyl and/or chlorine, $C_1$-$C_{10}$alkylthio or di($C_1$-$C_4$alkyl)amino, and $X_2$ is $C_1$-$C_{18}$alkyl, $C_3$-$C_{18}$alkenyl, $C_5$-$C_7$cycloalkyl, $C_1$-$C_4$alkyl-substituted $C_5$-$C_7$cycloalkyl, phenyl, $C_1$-$C_4$alkyl- and/or chlorine-substituted phenyl, $C_7$-$C_{12}$phenylalkyl or $C_7$-$C_{12}$phenylalkyl which is substituted on the phenyl ring by $C_1$-$C_4$alkyl and/or chlorine, and, if n is 1, Y is di($C_1$-$C_4$alkyl)thiocarbamoyl or a group of the formula IIa or IIb, and, if n is 2, Y is a group IIc or IId

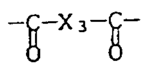 , 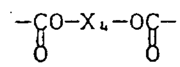

(IIc) (IId)

in which $X_3$ is $C_1$-$C_{12}$alkylene or phenylene and $X_4$ is $C_2$-$C_{12}$alkylene or $C_4$-$C_8$alkylene which is interrupted by 1 or 2 oxygen atoms, with the proviso that the radicals Y and $R_2$ are identical, if $R_2$ is di-($C_1$-$C_4$alkyl)thiocarbamoyl or a group of the formula IIa or IIb.

2. A composition according to claim 1, in which n is 1.

3. A composition according to claim 1, in which n is 1, $R_1$ is $C_1$-$C_4$alkyl or phenyl, $R_2$ is $C_1$-$C_{18}$alkyl, hydroxyl- or $C_1$-$C_6$alkyloxy-substituted $C_2$-$C_6$alkyl, $C_3$-$C_{18}$alkenyl, benzyl or benzoyl, Y is di($C_1$-$C_4$alkyl)thiocarbamoyl or a group IIa or IIb, $X_1$ is $C_1$-$C_{12}$alkyl, phenyl, dichlorophenyl, $C_1$-$C_{10}$alkylthio or di($C_1$-$C_4$alkyl)amino and $X_2$ is $C_1$-$C_{12}$alkyl.

4. A composition according to claim 1, in which n is 1, $R_1$ is methyl, $R_2$ is $C_1$-$C_4$alkyl, 2-hydroxyethyl, 2-methoxyethyl or allyl and Y is benzoyl or ($C_1$-$C_{12}$alkyl)oxycarbonyl.

5. A composition according to claim 1, in which $R_1$ is $C_1$-$C_4$alkyl or phenyl.

6. A composition according to claim 1, in which $R_1$ is methyl.

7. A composition according to claim 1, in which $R_2$ is $C_1$-$C_{18}$alkyl, hydroxyl- or $C_1$-$C_6$alkyloxy-substituted $C_2$-$C_6$alkyl, $C_3$-$C_{18}$alkenyl, benzyl or benzoyl.

8. A composition containing a) a chlorine-containing polymer and b) at least one compound of the formula I-A

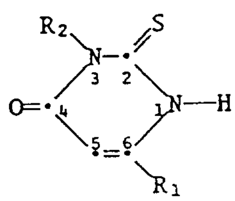

(I-A)

in which $R_1$ is $C_1$-$C_4$alkyl or phenyl and $R_2$ is $C_1$-$C_{18}$alkyl, hydroxyl- or $C_1$-$C_6$alkyloxy-substituted $C_2$-$C_6$alkyl, $C_3$-$C_{18}$alkenyl, benzyl or benzoyl, and as additional component c) an epoxy compound.

**9.** A composition according to claim 8, in which component b) is
3-methyl-6-n-propyl-2-thiouracil,
3-methyl-6-t-butyl-2-thiouracil,
3-ethyl-6-methyl-2-thiouracil,
3-allyl-6-methyl-2-thiouracil,
3-benzyl-6-methyl-2-thiouracil,
3-n-octyl-6-methyl-2-thiouracil,
3-(2'-hydroxyethyl)-6-methyl-2-thiouracil,
3-n-butyl-6-methyl-2-thiouracil,
3-i-octyl-6-methyl-2-thiouracil,
3-n-decyl-6-methyl-2-thiouracil,
3-n-dodecyl-6-methyl-2-thiouracil,
3-i-nonyl-6-methyl-2-thiouracil,
3-oleyl-6-methyl-2-thiouracil,
3-n-octadecyl-6-methyl-2-thiouracil,
3-i-butyl-6-methyl-2-thiouracil,
3-methyl-6-phenyl-2-thiouracil or
3-(2'-methoxyethyl)-6-methyl-2-thiouracil.

**10.** A composition according to claim 1 or 8, additionally containing at least one Me (II) carboxylate and/or Me (II) phenolate, in which Me (II) is Ba, Ca, Mg, Cd or Zn.

**11.** A composition according to claim 1 or 8, additionally containing at least one Me (II) carboxylate in which Me (II) is Ba, Ca, Mg or Zn.

**12.** A composition according to claim 1 containing as additional component c) an epoxy compound.

**13.** A composition according to claim 12, additionally containing at least one Me (II) carboxylate and/or Me (II) phenolate, in which Me (II) is Ba, Ca, Mg, Cd or Zn.

**14.** A composition according to claim 8, in which component b) is 3,6-dimethyl-2-thiouracil.

**15.** A compound of the formula I defined in claim 1.

**16.** A compound according to claim 15, in which n is 1, $R_1$ is $C_1$-$C_4$alkyl, $R_2$ is $C_1$-$C_4$alkyl, allyl, benzyl or benzoyl and Y is di($C_1$-$C_4$alkyl)thio-carbamoyl or a group IIa or IIb, $X_1$ is $C_1$-$C_{12}$alkyl, phenyl, dichlorophenyl, $C_1$-$C_{10}$alkylthio or di($C_1$-$C_4$alkyl)amino and $X_2$ is $C_1$-$C_{12}$alkyl.

**17.** A compound
3-methyl-6-n-propyl-2-thiouracil,
3-methyl-6-t-butyl-2-thiouracil,
3-(2'-hydroxyethyl)-6-methyl-2-thiouracil,
3-n-decyl-6-methyl-2-thiouracil,
3-n-dodecyl-6-methyl-2-thiouracil,
3-oleyl-6-methyl-2-thiouracil,
3-n-octadecyl-6-methyl-2-thiouracil
3-methyl-6-phenyl-2-thiouracil or
3-(2'-methoxyethyl)-6-methyl-2-thiouracil.

## Revendications

**1.** Composition contenant a) un polymère chloré et b) au moins un composé de formule I.

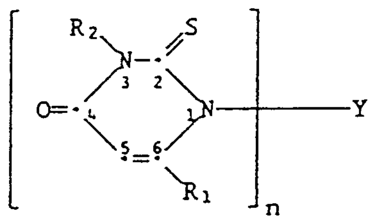

(I)

où n représente 1 ou 2, $R_1$ représente les alkyle en $C_1$-$C_{18}$, alcènyle en $C_3$-$C_{18}$, phényle, phénylalkyle en $C_7$-$C_{12}$ ou phénylalkyle en $C_7$-$C_{12}$ substitué sur le cycle de phényle par les alkyle en $C_1$-$C_4$ et/ou le chlore, $R_2$ représente un alkyle en $C_1$-$C_{18}$, un groupe

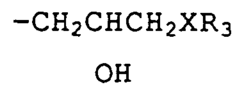

ou un alkyle en $C_2$-$C_{22}$ substitué par un hydroxy ou un groupe -$XR_3$, X étant l'oxygène ou le soufre et $R_3$ représentant les alkyle en $C_1$-$C_{18}$, phényle ou phénylalkyle en $C_7$-$C_{12}$, ou $R_2$ représente de plus les alcènyle en $C_3$-$C_{18}$, phénylalkyle en $C_7$-$C_{12}$, phénylalkyle en $C_7$-$C_{12}$ substitué sur le cycle de phényle par les alkyle en $C_1$-$C_4$ et/ou le chlore, di(alkyle en $C_1$-$C_4$)thiocarbamoyle ou un groupe de formule IIa ou IIb

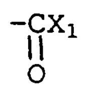 , 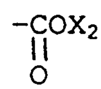

(IIa) (IIb)

$X_1$ représentant les alkyle en $C_1$-$C_{17}$, alcènyle en $C_3$-$C_{17}$, cycloalkyle en $C_5$-$C_7$, cycloalkyle en $C_5$-$C_7$ substitué par un alkyle en $C_1$-$C_4$, phényle, phényle substitué par les alkyle en $C_1$-$C_4$ et/ou le chlore, phénylalkyle en $C_7$-$C_{12}$, phénylalkyle en $C_7$-$C_{12}$ substitué sur le cycle de phényle par les alkyle en $C_1$-$C_4$ et/ou le chlore, alkylthio en $C_1$-$C_{10}$ ou di (alkyle en $C_1$-$C_4$ amino et $X_2$ représentant les alkyle en $C_1$-$C_{18}$, alcényle en $C_3$-$C_{18}$, cycloalkyle en $C_5$-$C_7$, cycloalkyle en $C_5$-$C_7$ substitué par un alkyle en $C_1$-$C_4$, phényle, phényle substitué par les alkyle en $C_1$-$C_4$ et/ou le chlore, phénylalkyle en $C_7$-$C_{12}$ ou phénylalkyle en $C_7$-$C_{12}$ substitué sur le cycle de phényle par les alkyle en $C_1$-$C_4$ et/ou le chlore, lorsque n vaut 1, Y représente un di(alkyle en $C_1$-$C_4$)thiocarbamoyle ou un groupe de formule IIa ou IIb, lorsque n vaut 2, Y représente un groupe IIc ou IId

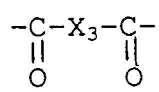 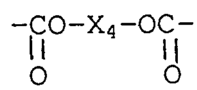

(IIc) (IId)

$X_3$ représentant les alkylène en $C_1$-$C_{12}$ ou phénylène et $X_4$ les alkylène en $C_2$-$C_{12}$ ou alkylène en $C_4$-$C_8$ interrompu par 1 ou 2 atomes d'oxygène à la condition que les radicaux Y et $R_2$ soient identiques lorsque $R_2$ représente un di(alkyle en $C_1$-$C_4$)thiocarbamoyle ou un groupe de formule IIa ou IIb.

**2.** Composition selon la revendication 1, dans laquelle n vaut 1.

**3.** Composition selon la revendication 1, où n vaut 1, $R_1$ représente les alkyle en $C_1$-$C_4$ ou phényle, $R_2$ représente les alkyle en $C_1$-$C_{18}$, alkyle en $C_2$-$C_6$ substitué par les hydroxy ou alkyloxy en $C_1$-$C_6$, alcényle en $C_3$-$C_{18}$, benzyle ou benzoyle, Y représente un di(alkyle en $C_1$-$C_4$)thiocarbamoyle ou un groupe IIa ou IIb, $X_1$ représente les alkyle en $C_1$-$C_{12}$, phényle, dichlorophényle, alkylthio en $C_1$-$C_{10}$ ou di (alkyle en $C_1$-$C_4$)amino et $X_2$ représente un alkyle en $C_1$-$C_{12}$.

**4.** Composition selon la revendication 1, où n vaut 1, $R_1$ représente le méthyle, $R_2$ les alkyle en $C_1$-$C_4$, 2-hydroxyéthyle, 2-méthoxyéthyle ou allyle et Y représente les benzoyle ou (alkyle en $C_1$-$C_{12}$) oxycarbonyle.

**5.** Composition selon la revendication 1 où $R_1$ représente les alkyle en $C_1$-$C_4$ ou phényle.

**6.** Composition selon la revendication 1, où $R_1$ représente le méthyle.

**7.** Composition selon la revendication 1, où $R_2$ représente les alkyle en $C_1$-$C_{18}$, alkyle en $C_2$-$C_6$ substitué par les hydroxy ou alkyloxy en $C_1$-$C_6$, alcényle en $C_3$-$C_{18}$, benzyle ou benzoyle.

**8.** Composition contenant a) un polymère chloré et b) au moins un composé de formule I-A :

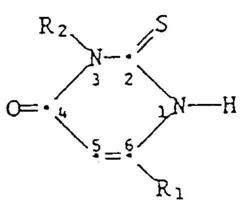

(I-A)

dans laquelle $R_1$ représente les alkyle en $C_1$-$C_4$ ou phényle et $R_2$ représente les alkyle en $C_1$-$C_{18}$, alkyle en $C_2$-$C_6$ substitué par les hydroxy ou alkyloxy en $C_1$-$C_6$, alcényle en $C_3$-$C_{18}$, benzyle ou benzoyle, et, en tant que composant supplémentaire c), un composé époxy.

**9.** Composition selon la revendication 8 dans laquelle le composant b) représente les
3-méthyl-6-n-propyl-2-thiouracile,
3-méthyl-6-t-butyl-2-thiouracile,
3-éthyl-6-méthyl-2-thiouracile,
3-allyl-6-méthyl-2-thiouracile,
3-benzyl-6-méthyl-2-thiouracile,
3-n-octyl-6-methyl-2-thiouracile,
3-(2'-hydroxyéthyl)-6-méthyl-2-thiouracile,
3-n-butyl-6-méthyl-2-thiouracile,
3-i-octyl-6-méthyl-2-thiouracile,
3-n-décyl-6-méthyl-2-thiouracile,
3-n-dodécyl-6-méthyl-2-thiouracile,
3-i-nonyl-6-méthyl-2-thiouracile,
3-oleyl-6-méthyl-2-thiouracile,
3-n-octadécyl-6-méthyl-2-thiouracile,
3-i-butyl-6-méthyl-2-thiouracile,
3-méthyl-6-phényl-2-thiouracile ou
3-(2'-méthoxyéthyl)-6-méthyl-2-thiouracile.

**10.** Composition selon la revendication 1 ou 8, contenant de plus au moins un carboxylate de Me(II) et/ou phénolate de Me(II), Me(II) signifiant Ba, Ca, Mg, Cd ou Zn.

**11.** Composition selon la revendication 1 ou 8, contenant de plus au moins un carboxylate de Me(II), Me(II) signifiant Ba, Ca, Mg, ou Zn.

**12.** Composition selon la revendication 1, contenant en tant que composant supplémentaire c) un composé époxy.

**13.** Composition selon la revendication 12, contenant de plus au moins un carboxylate de Me(II) et/ou phénolate de Me(II), Me(II) signifiant Ba, Ca, Mg, Cd ou Zn.

**14.** Composition selon la revendication 8, dans laquelle le composant b) représente le 3,6-diméthyl-2-thiouracile.

**15.** Composés de formule 1 définis dans la revendication 1.

**16.** Composés selon la revendication 15, où n vaut 1, $R_1$ représente un alkyle en $C_1$-$C_4$, $R_2$ représente les alkyle en $C_1$-$C_4$, allyle, benzyle ou benzoyle, et Y représente les di(alkyle en $C_1$-$C_4$)thiocarbamoyle ou un groupe IIa ou IIb, $X_1$ représente les alkyle en $C_1$-$C_{12}$, phényle, dichlorophényle, alkylthio en $C_1$-$C_{10}$ ou di(alkyle en $C_1$-$C_4$)amino et $X_2$ représente un alkyle en $C_1$-$C_{12}$.

**17.** Composés
3-méthyl-6-n-propyl-2-thiouracile,
3-méthyl-6-t-butyl-2-thiouracile,
3-(2'-hydroxyéthyl)-6-méthyl-2-thiouracile,
3-n-décyl-6-méthyl-2-thiouracile,
3-n-dodécyl-6-méthyl-2-thiouracile,
3-oleyl-6-méthyl-2-thiouracile,
3-n-octadécyl-6-méthyl-2-thiouracile,
3-méthyl-6-phényle-2-thiouracile et
3-(2'-méthoxyéthyl)-6-méthyl-2-thiouracile.